# EUROPÄISCHE PATENTSCHRIFT

(11) **EP 1 484 024 B1**
(45) Veröffentlichungstag und Bekanntmachung des Hinweises auf die Patenterteilung: **17.01.2007**
(21) Anmeldenummer: 04012935.5
(22) Anmeldetag: 01.06.2004
(51) Int. Cl.: A61B 17/28

(54) **Chirurgisches Instrument mit Instrumentengriff und Nullpunkteinstellung**
Surgical instrument with handgrip and zero setting arrangment
Instrument chirurgical avec poignée et un système de réglage de son point-zéro

(30) Priorität: 02.06.2003 DE 10324844
(43) Veröffentlichungstag der Anmeldung: 08.12.2004
(73) Patentinhaber: Tuebingen Scientific Medical GmbH, 72074 Tuebingen (DE)
(72) Erfinder: Braun, Marcus, 70563 Stuttgart-Vaihingen (DE)
(74) Vertreter: TBK-Patent

(56) Entgegenhaltungen:
- DE-A1- 10 036 108
- US-A- 5 354 311
- US-A- 5 549 637
- US-A- 5 752 973

## Beschreibung

Die vorliegende Erfindung betrifft ein chirugisches Instrument für die minimal invasive Chirurgie gemäß dem Oberbegriff des Patentanspruchs 1.

DE 100 36 108 offenbart ein chirugisches Instrument gemäß dem Oberbegriff des Anspruch 1. Dieses besteht im wesentlichen aus einem Rohrschaft, an dessen einem proximalen Ende ein Instrumentengriff angeordnet ist, mittels dem ein an dem gegenüberliegenden distalen Ende des Rohrschafts angeordneter Instrumentenkopf über Getriebezüge betätigbar ist. Der Instrumentenkopf läßt sich bezüglich des Rohrschafts verschwenken bzw. neigen und trägt zudem einen drehbar im Instrumentenkopf gelageren Effektor in Form einer Art Zange oder Greifer, deren eines Zangenst ück schwenkbar am Effektor gelagert und mittels des Instrumentengriffs ebenfalls betätigbar ist.

Konkreter ausgedrückt ermöglichen die Getriebezüge, dass zumindest eine erste Bewegung des Instrumentengriffs, gemäß diesem Stand der Technik auslösbar durch die Handrotation einer Bedienerperson, in eine Rotation des Effektors unter einem bestimmten Übersetzungsverhältnis zu dieser Betätigungsbewegung transformiert wird. Hierdurch ist es möglich, den Effektor trotz der relativ eingeschränkten Bewegungsmöglichkeit einer menschlichen Hand um beispielsweise bis zu 300° zu drehen und damit komplizierte Bewegungsabläufe ohne Umgreifen am Handgriff zu realisieren. Des weiteren wird eine zweite Bewegung des Instrumentengriffs, beispielsweise dessen Abwinkeln bezüglich des Rohrschafts, in eine Neigungsbewegung des Instrumentenkopfs umgesetzt.

Die Getriebezüge innerhalb des Instrumentengriffs und des Rohrschafts sind derart ausgelegt, dass eine weitestgehend entkoppelte Betätigung jeder einzelnen Bewegung des Instrumentenkopfs sowie des Effektors ermöglicht wird. Allderdings sind derartige Getriebe zwangsläufig äußerst kompliziert und benötigen demzufolge auch ausreichend Bauraum. Darüber hinaus ist eine vollständige Entkopplung der einzelnen Bewegungen nicht gänzlich gewährleistet.

Auch hat es sich gezeigt, dass insbesondere bei einer Handrotation zum Drehen des im Instrumentenkopf gelagerten Effektors, bedingt durch die natürliche Handkonstruktion und der daraus resultierenden Bewegungsabläufe, d.h. unabhängig davon, ob die Getriebezüge des chirurgischen Instrumentes tatsächlich vollständig entkoppelt sind oder nicht, gleichzeitig auch ein geringfügiges Abwinkeln der Instrumentenspitze und darüber hinaus ein Abkippen des gesamten Instrumentenschafts bewirkt wird, wodurch sich die Handhabung des Instuments erheblich erschwert.

Angesichts dieses Stands der Technik ist es eine Aufgabe der vorliegenden Erfindung, ein chirugisches Instrument dieser Gattung zu schaffen, bei welchem Bewegungen eines Instrumentenkopfs sowie eines Effektors weitestgehend unabhängig von natürlichen Gegebenheiten einer natürlichen Handkonstruktion voneinander entkoppelt über einen Instrumentengriff ausführbar sind.

Diese Aufgabe wird durch ein chirugisches Instrument mit den Merkmalen gemäß dem Patentanspruch 1 gelöst.

Grundsätzlich sind die einzelnen Freiheitsgrade sowie die Bewegungsbereiche einer menschlichen Hand aufgrund ihrer Konstruktion in einem eingeschränkten Bereich vorgegeben und weichen individuell auch nur geringfügig voneinander ab. Der Einsatz eines gattungsgemäßen chirurgischen Instruments kann jedoch oftmals keine Rücksicht auf kinematische Beschränkungen der menschlichen Hand nehmen. In anderen Worten ausgedrückt erfordert ein optimaler Einsatz des chirurgischen Instruments ein Handstellung oder ein Position des Chirurgen, welche ggf. unnatürlich ist. Dies führt dazu, dass solche Stellungen nicht lange und nur mit großem Kraftaufwand gehalten werden können, so dass darüber hinaus in solchen Stellungen ein exaktes Arbeiten mit dem chirurgischen Instrument nur sehr schwer möglich ist.

Auf diesen Überlegungen basierend besteht der Kern der Erfindung nunmehr darin, den Instrumentengriff über eine Kupplung mit dem Rohrschaft schwenkbar zu verbinden oder besser ausgedrückt, in den Getriebezug zur Übertragung einer Schwenkbewegung des Instrumentengriffs auf den Instrumentenkopf für dessen Abwinklung eine Kupplung zwischenzufügen, die eine zumindest über einen vorbestimmten Bereich individuelle Nullpunkteinstellung erlaubt, wobei der Nullpunkt eine Relativposition des Instrumentengriffs bezüglich des Rohrschafts ist, in der der Instrumentenkopf eine vorbestimmte Abwinkelposition, vorzugsweise die maximale oder minimale Abwinkelposition (Anschlagsposition) bezüglich des Rohrschafts einnimmt.

Durch diese technische Maßnahme kann der Instrumentengriff bzw. dessen Relativlage zum Rohrschaft in Abhängigkeit von der Einsatzlage des Instruments so ausgerichtet werden, dass der Chirurg den Instrumentengriff im wesentlichen innerhalb des natürlichen Bewegungsbereichs seiner Hand ergreifen und betätigen kann. Ein ermüdungsfreies und exaktes Arbeiten mit dem erfindungsgemäßen chirurgischen Instrument wird somit möglich.

Vorteilhaft ist es, die Kupplung selbstauslösend auszubilden. D. h. ein solche Kupplung rückt selbsttätig bei Überschreiben eines vorbestimmten Drehmoments aus bzw. überträgt nur dieses vorbestimmte Drehmoment und beginnt darüber hinaus zu gleiten.

Eine vorteilhafte Möglichkeit der konstruktiven Augestaltung einer selbstauslösenden Kupplung ist die Ausbildung einer Rutschkupplung, welche prinzipiell zwei gegeneinander gedrückte Drehmomentübertragungselemente vorsieht, welche ab einem bestimmten Drehmoment aneinander abgleiten. Konkret sind die Drehmomentübertragungselemente so gegeneinander vorgespannt, dass sie ein Drehmoment im Rahmen eines herkömmlichen Einsatzes des Instruments durch entsprechenden Reibkontakt übertragen können, welches jedoch für den Fall, dass der Instrumentenkopf sich in seiner maximalen oder minimalen Abwinkelungs-Anschlagsposition befindet, durch weiteres Schwenken des Instrumentengriffs überschritten und damit die Relativlage beider Drehmomentübertragungselemente verändert wird.

Alternativ hierzu ist es natürlich auch möglich, zwei Drehmomentübertragungselemente der Kupplung manuell für eine Nullpunkteinstellung außer Eingriff zu bringen, wobei in diesem Fall jedoch die Einhaltung der vorbestimmten Position des Instrumentenkopfs für eine gezielte Einstellung des Nullpunkts sichergestellt sein muß.

Als eine weitere Alternative für eine selbstauslösende Kupplung kann kann auch ein Eingriffselement bspielsweise in Form zumindest eines Mitnehmerzahns oder zumindest einer Kugel vorgesehen sein, das an einem der Drehmomentübertragungselemente gelagert und gegen das andere Drehmomentübertragungselement federvorgespannt ist und in eine Ausnehmung oder Hinterschneidung eingreift. Hierdurch wird eine Formschlußverbindung geschaffen, welche bei Überschreiten eines durch die Federvorspannung bestimmten Drehmoments gelöst wird und sich die beiden Drehmomentübertragungselements relativ verdrehen.

Eine weitere vorteilhafte Ausgestaltung der Erfindung sieht vor, die Kupplung im Bereich der Anlenkstelle zwischen dem Instrumentengriff und dem Rohrschaft anzuordnen. Dies hat den Vorteil, dass bei der Dimensionierung der Kupplung geringere räumliche Beschränkungen herrschen, da sich dieser Abschnitt des Instruments grundsätzlich immer außerhalb des zu operierenden Körpers befindet und zudem dieser Bereich für eine sichere Handhabung ohnehin groß gebaut ist entsprechend den Abmessungen einer menschlichen Hand.

Weitere vorteilhafte Ausgestaltungen der Erfindung sind Gegenstand der Unteransprüche.

Die Erfindung wird nachstehend anhand eines bevorzugten Ausführungsbeispiels unter Bezugnahme auf die begleitenden Zeichnungen näher erläutert.
Fig. 1 zeigt eine Perspektivenansicht eines chirurgischen Instruments gemäß einem bevorzugten Ausführungsbeispiels der Erfindung,
Fig. 2 zeigt einen ersten Getriebezug für ein Verschwenken eines Instrumentenkopfs mittels eines Instrumentengriffs sowie insbesondere ein Nullpunkt-Einstellvorrichtung im Anlenkungsbereich des Instrumentengriffs,
Fig. 3 zeigt eine Teilschnittansicht des ersten Getriebezugs im Schwenkbereich des Instrumentenkopfs,
Fig. 4 zeigt einen zweiten Getriebezug für ein Rotieren des Instrumentenkopfs mittels des Instrumentengriffs,
Fig. 5 zeigt eine Teilschnittansicht des zweiten Getriebezugs im Schwenkbereich des Instrumentenkopfs und
Fig. 6a-6c zeigen Schnittdarstellungen eines dritten Getriebezugs im Schwenkbereich des Instrumentenkopfs zur Betätigung einer am Instrumentenkopf gelagerten Zange.

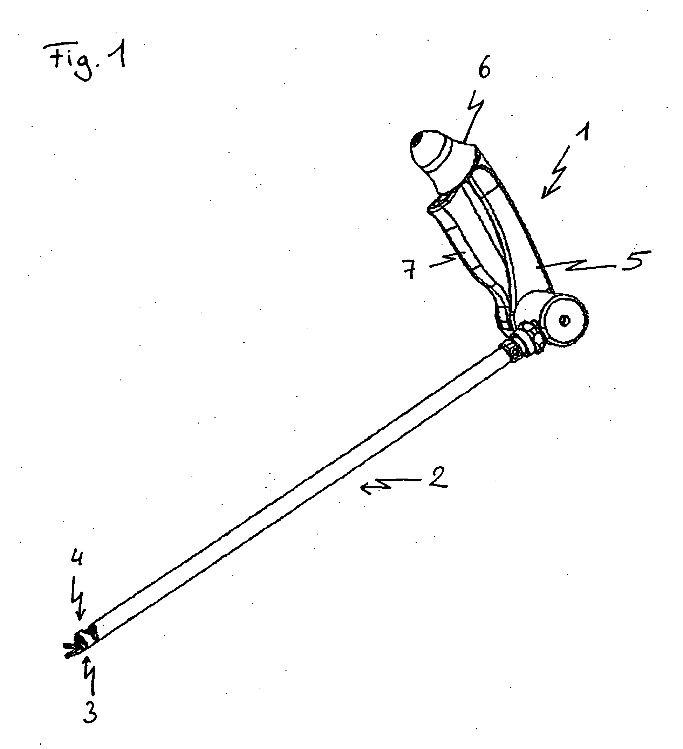

In der Fig. 1 ist ein vollständiges chirurgisches Instrument gemäß einem bevorzugten Ausführungsbeispiel der Erfindung in einer Perspektivenansicht dargestellt. Das erfindungsgemäße chirurgische Instrument hat demzufolge einen multifunktionalen Instrumentengriff 1, der an einem proximalen Ende oder Endabschnitt eines vorzugsweise aus nichtrostendem Stahl, einer Stahllegierung oder einem Kunststoffmaterial gefertigten Rohrschafts 2 angeordnet ist, sowie einem mit einem Effektor 3 bestückten oder bestückbaren Instrumentenkopf 4, der an dem anderen, distalen Ende des Rohrschafts 2 vorgesehen ist.

Generell ist der Instrumentenkopf 4 an dem betreffenden Rohrschaftende so gelagert, dass er bezüglich des Rohrschafts 2 verschwenkt bzw. abgewinkelt werden kann, wohingegen der Effektor 3 in jeder Abwinkelposition des Instrumentenkopfs 4 um dessen Längsachse gedreht bzw. rotiert werden kann, wobei die beiden vorstehend genannten Bewegungen mittels des Instrumentengriffs 1 ausführbar sind. Hierzu sind am Instrumentengriff 1 eine Anzahl von Handhaben und/oder Betätigungsmechanismen vorgesehen, die über entsprechende Getriebezüge innerhalb des Instrumentengriffs 1 sowie des Rohrschafts 2 mit dem Instrumentenkopf 4 bzw. dem Effektor 3 wirkverbunden sind, um die einzelnen Bewegungen des Instrumentenkopfs 4 und des Effektors 3 unabhängig voneinander, d.h. entkoppelt ausführen zu können.

Konkret besteht der Instrumentengriff 1 aus einem ergomisch geformten Griffstück 5, das schwenk- bzw. neigbar am Rohrschaft 2 montiert ist und an dem eine erste Handhabe 6, vorliegend vorzugsweise in Form eines Drehknopfs sowie eine zweite Handhabe 7, vorliegend vorzugsweise in Form eines Griffhebels gelagert sind. Somit besitzt der Instrumentengriff 1 gemäß dem bevorzugten Ausführungsbeispiel der vorliegenden Erfindung insgesamt drei Betätigungsmechanismen für drei unabhängige Bewegungen des bzw. am Instrumentenkopf 4. An dieser Stelle sei ausdrücklich darauf hingewiesen, dass der Instrumentengriff 1 auch weniger Betätigungsmöglichkeiten aufweisen kann, etwa jeweils nur einen Betätigungsmechanismus für ein Verschwenken des Instrumentenkopfs 4 und Drehen des Effektors 3.

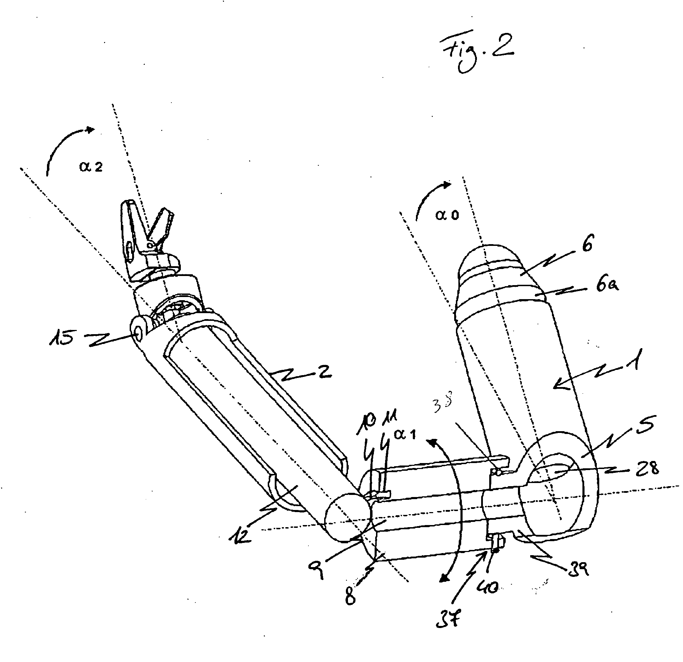

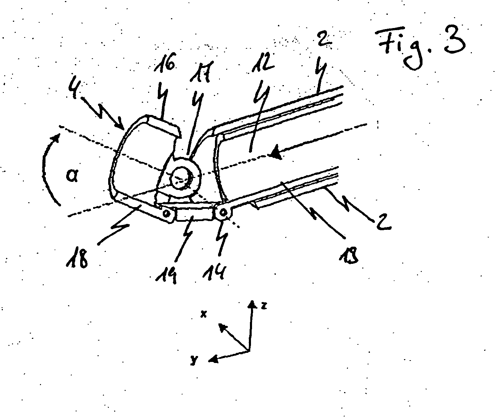

Der äußere Aufbau des Instrumentengriffs 1 insbesondere mit Bezug auf den Betätigungsmechanismus für ein Verschwenken bzw. Abwinkeln des Instrumentenkopfs 4 sowie den zugehörigen Abwinklungs-Getriebezug ist in den Fig. 2 und 3 dargestellt.

Das in Fig. 2 idealisiert dargestellte Griffstück 5 ist über ein mit dem Griffstück 5 über eine nachfolgend noch beschriebene Kupplung 37 verbundenes Kulissenelement 8 in Form einer Drehwelle oder Drehscheibe mit dem Rohrschaft 2 schwenkbar verbunden. Hierbei ist die Drehwelle 8 vorzugsweise senkrecht zum Rohrschaft 2 sowie zum Griffstück 5 ausgerichtet und beabstandet das Griffstück 5 vom Rohrschaft 2 derart, dass dieses im wesentlichen parallel zum Rohrschaft 2 an diesem vorbei verschwenkt werden kann.

An dem Griffstück 5 selbst ist eine Art rohr- oder hülsenförmiger Anschlusssockel 39 an dessen proximalem Endabschnitt fixiert, der sich im wesentlichen senkrecht, d.h. in Radialrichtung vom Griffstück 5 weg erstreckt. Der rohrförmige Anschlusssockel 39 ist an seinem freien Ende mit einer äußeren Gleitfläche (nicht näher dargestellt) ausgebildet, auf die ein offener Federring 38 unter Spannung aufgesetzt ist.

Die Drehwelle 8 sitzt an ihrem dem Griffstück 5 zugewandten Endabschnitt auf dem Anschlusssockel 39 drehbar auf. Hierfür ist die Drehwelle 8 innenseitig über eine vorbestimmte axiale Länge auf einen Innendruchmesser aufgebohrt, der soviel größer ist als der Außendurchmesser des Anschlusssockels 39, dass zwischen diesem und der Drehwelle 8 zumindest eine Spielpassung entsteht. Hirdurch wird an der aufgebohrten Innwandung der Drehwelle 8 eine Umfangsfläche mit vorbesimmtem Radius ausgebildet, aus der ein radial verlaufender Stift 40 ragt, der durch eine radiale Durchgangsbohrung von der Mantelseite der Drehwelle 8 nach Innen eingesteckt ist.

Bei der Montage der Drehwelle 8 wird diese auf den Anschlusssockel 39 aufgesteckt, bis dass der Federing 38 auf oder an der inneren Umfangsgleitfläche der Drehwelle 8 positioniert ist. Der Stift 40 ragt dabei in den vom offenen Federing 38 gebildeten Radialspalt und bildet somit eine Formschlußverbindung zwischen der Drehwelle 8 und dem Federring 38, welcher wiederum eine Reibschlußverbindung mit dem Anschlusssockel 39 eingeht. An dieser Stelle sei jedoch explizit darauf hingewiesen, dass die vorstehende Kupplung auch in kinematisch umgekehrter Weise ausgebildet sein kann, d.h. der Federing 38 reibschlüssing in der Drehwelle 8 sitzen und über einen im Anschlussstutzen steckenden Stift mit diesem eine Formschlussverbindung eingehen kann.

Wird der Instrumentengriff 1 nunmehr um seine Schwenkachse (entspricht der Mittelachse der Drehwelle 8) geschwenkt, wird die Schwenkbewegung über den reibschlüssig auf der Umfangsgleitfläche des Anschlusssockels 39 oder der Drehwelle 8 sitzenden Federings 38 auf den in den Federingspalt ragenden Stift 40 und damit auf die Drehwelle 8 oder den Anschlussstutzen 39 übertragen, welche(r) sich somit einstückig mit dem Instrumentengriff 1 mitdreht. Das maximal übertragbare Drehmoment bestimmt sich dabei durch die Vorspannkraft, mit welcher der Federing 38 auf die Umfangsgleitfläche aufgesetzt ist. Sobald dieses Drehmoment überschritten wird, beginnt der Federing 38 auf der Umfangsgleitfläche zu gleiten, wodurch sich die relative Drehposition zwischen dem Instrumentengriff 1 und der Drehwelle 8 verändert.

An dieser Stelle sei darauf hingewiesen, dass der Federring 38 natürlich auch in entsprechende Umfangsführungen beispielsweise in Form von Innen- und/oder Außennuten an der Drehwelle 8 und/oder dem Anschlusssockel 39 eingeschnappt sein kann, um eine axiale Führung zu haben.

Die Drehwelle 8, welche einen zentralen Durchgangskanal 9 zur Aufnahme nachfolgend noch beschriebener Getriebeteile ausbildet, ist an ihrer einen, dem Rohrschaft 2 zugewandten Stirnseite mit einer Kulissenführung 10 in Form einer nockenförmigen Nut ausgebildet, in die ein Mitnehmerstift 11 eingreift, welcher an einem im Rohrschaft 2 gelagerten, axial verschiebbaren Schubrohr 12 befestigt ist. Die Nut 10 ist dabei derart ausgebildet, dass bei einer Drehbewegung der Drehwelle 8 durch entsprechendes Schwenken des Griffstücks 1 der Mitnehmerstift 11 in der Nut 10 entlang gleitet und dabei eine Zwangs-Ausgleichsbewegung in Längsrichtung des Rohrschafts 2 ausführt, die auf das Schubrohr 12 übertragen wird und je nach Drehrichtung der Drehwelle 8 zu einer Hin- und Herbewegung des Schubrohrs 12 innerhalb des Rohrschafts 2 führt.

Zusammenhehalten wird der vorstehend beschriebene Aufbau schließlich durch eine Nabe in Form eines Nabenbolzens (nicht näher gezeigt), der am Rohrschaft fixiert ist und die Drehwelle 8 wie auch das Griffstück 5 in dessen Schwenkachse durchdringt und mittels einer ebenfalls nicht weiter dargestellen Mutter gekontert ist.

Der dem Kulissenelement 8 abgewandte, distale Endabschnitt des Schubrohrs 12 ist mit einer längs sich ersteckenden Lasche 13 ausgebildet, die über das distale Ende des Schubrohrs 12 vorsteht und an ihrem freien Endabschnitt ein Scharnier bzw. Scharnierösen 14 bildet. Des weiteren ist die Stirnseite des Rohrschafts 2 an seinem distalen Endabschnitt in einem Winkel von vorzugsweise 45° abgeschrägt und mit seitlichen Gelenkösen 15 ausgebildet, an denen der Instrumentenkopf 4 schwenkbar über Gelenkzapfen oder Stifte angelenkt ist. Dieser besteht ebenfalls aus einem Rohrstück 16, dessen eine Stirnseite, an der Anlenkösen 17 für ein Verbinden mit dem Rohrschaft 2 bzw. dessen Gelenkösen 15 ausgebildet sind, ebenfalls in einem Winkel von vorzugsweise 45° abgeschrägt ist und zwar derart, dass sich nach einem Anlenken des Instrumentenkopfs 4 am Rohrschaft 2 die beiden vorstehend genannten Schrägen ergänzen und ein Abwinkeln des Rohrstücks 16 bezüglich des Rohrschafts 2 um ca. 90° vorzugsweise 70° ermöglichen.

Des weiteren ist an der abgeschrägten Stirnseite des Rohrstücks 16 ein Scharnier bzw. Scharnierösen 18 ausgebildet. An den schubrohrseitigen sowie den rohrstückseitigen Scharnierösen 14; 18 ist ein Kipphebel 19 jeweils anscharniert, welcher folglich zur Schwenkachse des Instrumentenkopfs 4 radial nach außen versetzt ist und eine axiale Translationsbewegung des Schubrohrs 12 auf das Rohrstück 16 überträgt, wodurch dieses um dessen Schwenkachse geschwenkt wird.

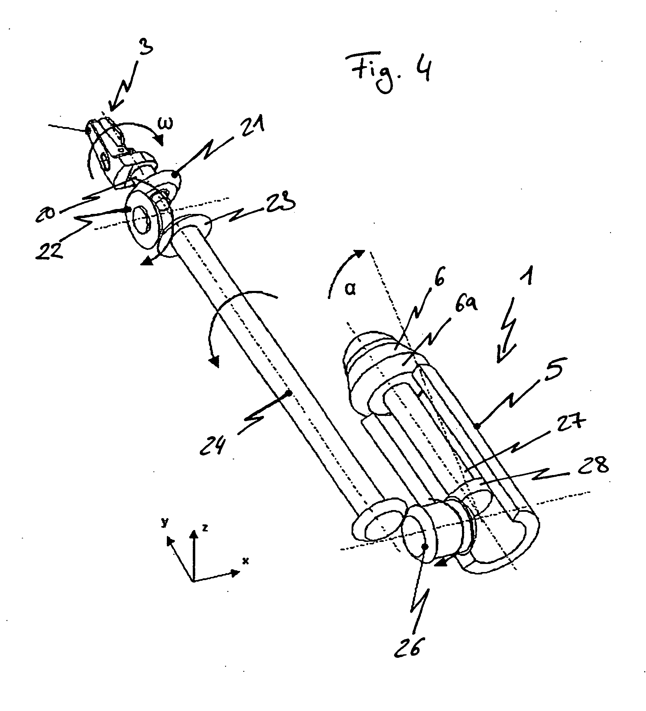

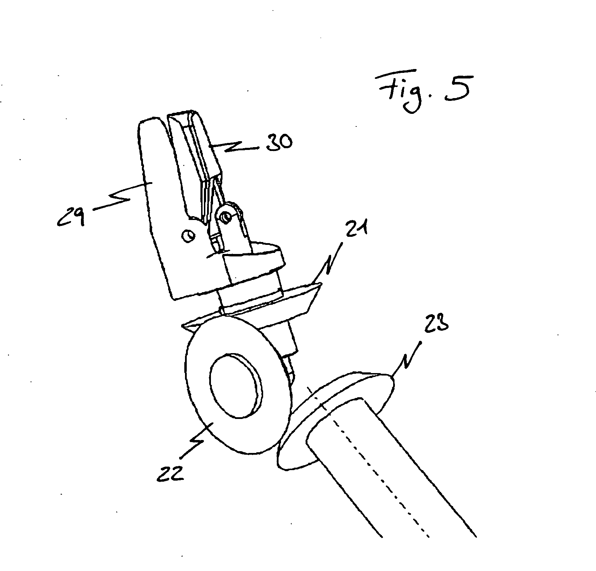

Im nachfolgenden wird anhand der Fig. 4 und 5 der Betätigungsmechanismus für eine Rotation des im Instrumentenkopf 4 gelagerten Effektors 3 sowie der zugehörige Rotations-Getriebezug beschrieben.

Wie noch aus der Fig. 2 zu entnehmen ist, stellt das vorstehend genannte Rohrstück 16 des Instrumentenkopfs 4 gleichzeitig ein Gehäuse bzw. eine Aufnahme für den Effektor 3 dar. Unabhängig davon, um welche Art Effektor es sich dabei handelt, also unabhängig davon, ob als Effektor beispielsweise ein Nadelhalter, ein Greifer, eine Zange oder Schere zum Einsatz kommt, besitzt dieser eine vorzugsweise hohle Rotationsachse 20, die in das Rohrstück 16 des Instrumentenkopfs 4 drehbar eingesetzt und gegen eine Axialbewegung gesichert ist. Die Länge dieser Rotationsachse 20 ist dabei so gewählt, dass diese in etwa im Bereich der Schwenkachse des Instrumentenkopfs 4 endet und an ihrem freien, zu dieser Schwenkachse ragenden Ende mit einem Abtriebs-Stirnrad 21 versehen ist, das drehfest an der Rotationsachse 20 des Effektors 3 befestigt ist. Insbesondere in der Fig. 2 ist die Schwenkachse des Instrumentenkopfs 4 durch eine Strich-Linie durch die Ösen 15 dargestellt.

Wie aus der Fig. 5 ferner zu entnehmen ist, befindet sich auf der Schwenkachse des Instrumentenkopfs 4 ein Drehmoment-Übertragungsstirnrad 22, welches an einem der beiden nicht weiter dargestellten Schwenkzapfen des Instrumentenkopfs 4, welche die idealisiert dargestelle Schwenkachse bilden, drehbar gelagert und mit dem Abtriebs-Stirnrad 21 in Kämmeingriff ist. Das Drehmoment-Übertragungsstirnrad 22 ist wiederum mit einem Antriebs-Stirnrad 23 in Kämmeingriff, welches auf einer innerhalb des Schubrohrs 12 (in den Fig. 4 und 5 nicht dargestellt) drehbar geführten Antriebswelle 24 drehfest montiert ist, wie dies insbesondere in der Fig. 4 gezeigt ist. An einem dem Antriebs-Stirnrad 23 gegenüberliegenden Ende der Antriebswelle 24 ist gemäß der Fig. 4 ein weiteres Drehmoment-Einleitungsstirnrad 25 drehfest angeordnet, welches mit einer Zahnradwelle 26 in Kämmeingriff ist, die in dem innerhalb des Kulissenelements 8 ausgebildeten zentralen Durchgangskanal 9 gelagert ist.

Das Kulissenelement 8 ist in der Fig. 4 nicht weiter dargestellt.

Die Zahnradwelle 26 ist schließlich mit einer Betätigungswelle 27, bzw. einem daran befestigten Stirnrad 28 innerhalb des Griffstücks 5 in Kämmeingriff, welche mit der einen Handhabe, vorliegend dem Drehknopf 6, fest verbunden ist.

Wie insbesondere aus der Fig. 4 zu entnehmen ist, bildet der Drehknopf 6 die distale Spitze oder das äußere Ende des Instrumentengriffs 1. Er ist dabei drehfest mit der Betätigungswelle 27 verbunden, welche sich im wesentlichen entlang der Längsachse des Griffstücks 5 erstreckt. Der Drehknopf 6 hat einen zum in der Fig. 4 idealisiert als Hülse dargestellten Griffstück 5 zugewandten hinteren Randabschnitt, der gleitend am Griffstück 5 anliegt und damit das Griffstück 5 an seinem distalen Ende abdichtet.

Bei einer Betätigung des Drehknopfs 6 wird dessen Drehbewegung über die Betätigungswelle 27 innerhalb des Griffstücks 5, die Zahnradwelle 26, die daran sich anschließende Antriebswelle 24 innerhalb des Schubrohrs 12 sowie das Übertragungsstirnrad 22 auf den Effektor 3 übertragen und dieser gedreht. Der Drehknopf 6 wird dabei in vorteilhafter Weise mit den Fingern insbesondere mit Daumen und Zeigefinder der Bedienerhand betätigt, während das Griffstück 5 in Hand gehalten wird. Es ist somit möglich, jede beliebige Rotation am Effektor 3 zu erzeugen, ohne dass der Bediener am Griffstück 5 selbst umgreifen muss. Die Finger einer menschlichen Hand sind in der Lage, sehr feinmotorisch zu arbeit und auch die Fingerspitzen sind mit einer Vielzahl von Nervenenden versehen, die ein ausgeprägtes taktiles Gefühl ermöglichen. Demnach sollten Bewegungen, die eine hohe Genauigkeit erfordern, mit den Fingern ausgeführt werden. Es hat sich gezeigt, dass die Drehbewegung des Effektors 3 eine solche ist und daher erfindungsgemäß durch den Drehknopf 6 ausgelöst wird, ohne dass in vorteilhafter Weise durch das Drehen des Knopfs 6 für eine Rotation des Effektors 3 eine Bewegung auf den Rohrschaft 2 übertragen wird. Vielmehr kann die Hand einer Bedienerperson ruhig gehalten werden.

An dieser Stelle sei ferner noch darauf hingewiesen, dass die Antriebswelle 24 sowie das Schubrohr 12 in Axialrichtung relativ beweglich zueinander gelagert sind. D.h. eine durch ein Verschwenken des Instrumentengriffs 1 ausgelöste Drehung des Kulissenelements 8 bewirkt zwar eine Translationsbewegung des Schubrohrs 12. Gleichzeitig wird jedoch die Antriebswelle 24 in Position, d.h. in Kämmeingriff mit der Zahnradwelle 26 gehalten, wodurch das Schubrohr 12 eine axiale Relativbewegung zum Rohrschaft 2 wie auch zur Antriebswelle 24 ausführt.

Schließlich wird nachfolgend der Betätigungsmechanismus für den Effektor 3, d.h. dessen Funktionen selbst sowie den zugehörigen Effektor-Getriebezug anhand der Fig. 5 und 6a-6c beschrieben.

Gemäß der Fig. 5 ist der Effektor 3 in dem vorliegenden Ausführungsbeispiel der Erfindung als eine Zange mit einer feststehenden sowie einer bewegbaren, d.h. schwenkbaren Zangenbacke 29; 30 ausgebildet. Die feststehende Zangenbacke 29 bildet zusammen mit der Rotationswelle 20 des Effektors 3 eine Einheit und ist vorzugsweise einstückig mit der Rotationswelle 20 ausgebildet, wohingegen die bewegbare Zangenbacke 30 an einem Ende schwenkbar an der feststehenden Zangenbacke 29 angelenkt ist.

Die bewegbare Zangenbacke 30 bildet eine Anlenkstelle 31 für einen Schubbolzen 32, der innerhalb der Rotationswelle 20 relativ verschiebbar gelagert ist, sodass durch dessen Axialverschiebung eine Schwenkbewegung der bewegbaren Zangenbacke 30 mit möglichst großer Übersetzung bewirkt wird. Der Schubbolzen 32 ist, wie dies insbesondere in den Fig. 6a - 6c dargestellt wird, mittels einer Feder 33 axial in Öffnungsrichtung der Zange vorgespannt, die innerhalb der Rotationsachse 20 den Schubbolzen 32 umgibt. Hierfür bildet der Schubbolzen 32 einen Wellenabsatz, an dem sich die Vorspannfeder 33 an ihrem einen Ende abstützt. Das andere Ende der Vorspannfeder 33 ist gegen die feststehende Zangenbacke 29 abgestützt. Ein aus der Rotationsachse 20 in Richtung zur Schwenkachse des Instrumentenkopfs 4 herausragendes Endstück 34 des Schubbolzens 32 ist kugelkopfförmig ausgebildet, wobei der Radius des Kugelkopfs 34 vorliegend vorzugsweise ca. 2.5 mm beträgt.

Die vorstehend genannte Antriebswelle 24 für das rotorische Antreiben des im Instrumentenkopf 3 gelagerten Effektors 3 ist mit einer im wesentlichen durchgehenden Axialbohrung (nicht weiter gezeigt) versehen. In dieser Axialbohrung ist ein Schubstab 35 axialverschieblich, sowie relativ zur Antriebswelle 24 drehbar geführt, dessen dem Schubbolzen 32 zugewandte Stirnseite entsprechend den Abschrägungen der rohrschaftseitigen wie auch schubrohrseiten distalen Stirnseiten d.h. vorzugsweise 45° in die selbe Richtung abgeschrägt ist. Der Schubbolzen 32 ist mittels der Feder 33 gegen diese abgeschrägte Stirnfläche des Schubstabs 35 vorgespannt und kommt mit dieser in Anlage. Dabei ist die Anlagefläche zwischen dem Schubstab 35 und dem Schubbolzen 32 aufgrund der vorstehend beschriebenden Kugelkopfform des Bolzens 32 im wesentlichen punktförmig und zwar unabhängig von dem Abwinkelungsgrad des Instrumentenkopfs 4 sowie unabhängig von der Rotationsposition des Effektors 3.

Wie aus der Fig. 6a zu erkennen ist, sind der Schubbolzen 32 wie auch der Schubstab 35 für den Fall, dass die Abwinkelung des Instrumentenkopfs 4 bezüglich des Rohrschafts 2 im wesentlichen 0° beträgt, axial zueinander ausgerichtet. Darüber hinaus ist der Schubbolzen 32 in dieser Stellung des Instrumentenkopfs 4 so positioniert, dass der Mittelpunkt des Kugelkopfs 34 des Schubbolzens 32 in etwa in der Schwenkachse des Instrumentenkopfs 4 liegt.

Der Schubstab 35 ist an seinem proximalen Ende über ein nicht im Einzelnen gezeigtes Getriebe 36 mit dem Betätigungshebel 7 verbunden, der, wie Eingangs dieser Beschreibung bereits kurz ausgeführt wurde, schwenkbar am Griffstück 5 gelagert ist.

Die Funktionsweise des erfindungsgemäßen chirurgischen Instruments wird nachfolgend im Einzelnen beschrieben.

Eine Rotation des im Instrumentenkopf 4 gelagerten Effektors 3 erfolgt durch eine Betätigung des an einem Ende des Griffstücks 5 gelagerten Drehkopfs 6, wobei der Drehknopf 6 wie vorstehend bereits ausgeführt wurde, soweit um seine Drehachse gedreht werden kann, dass eine ca. 360° Rotation am Effektor 3 realisiert wird, ohne dass am Griffstück 5 notwendiger Weise umgegriffen werden muss. Diese Drehbewegung wird über die Betätigungswelle 27 auf die Zahnradwelle 26 übertragen, welche wiederum ihre Drehbewegung auf die innerhalb des Schubrohrs 12 verlaufende Antriebswelle 24 weiter gibt. Die Drehbewegung der Antriebswelle 24 bewirkt eine Drehbewegung des Übertragungsstirnrads 22, welches die Schwenkachse des Instrumentenkopfs 4 quasi überbrückt und damit eine Rotationsbewegung des Effektors 3 innerhalb des Rohrstücks 16 des Instrumentenkopfs 4 um die Rohrstückachse auslöst.

Um eine Abwinkelung, d.h. eine Schwenkbewegung des Instrumentenkopfs 4 und damit des Effektors 3 zu bewirken, muß gemäß dem vorliegenden Ausführungsbeispiel das gesamte Griffstück 5 um die Längsachse des Kulissenelements bzw. die Drehwelle 8 geschwenkt werden. In anderen Worten ausgedrückt, bewirkt eine Schwenkbewegung des Griffstücks 5 bezüglich des Rohrschafts 2 eine Drehbewegung des über die Kupplung 37 mit dem Griffstück 5 in Rotationsrichtung reibschlüssig verbundenen Kulissenelements 8. Gleichzeitig jedoch wird aufgrund der Tatsache, dass durch den Kämmeingriff zwischen der Betätigungsachse 27 und der Zahnradwelle 26 eine Art Selbsthemmung durch Reibung (Wirkungsgrad des Getriebes) entsteht, welche ggf. durch leichtes Halten des Betätigungsknopfs 6 sowie durch die Haftreibung zwischen Betätigungsknopf 6 und Griffstück 1 noch unterstützt wird, die Zahnradwelle 26 mit dem Kulissenelement 8 mit gedreht.

Die Drehung des Kulissenelements 8 wird über die Kulisse bzw. Nut 10 an der Stirnseite des Elements 8 sowie den Mitnehmerzapfen 11 in eine Axialbewegung des Schubrohrs 12 übertragen, die über den anscharnierten Kipphebel 19 zu einer Schwenkbewegung des Instrumentenkopfs 4 um dessen Schwenkachse transformiert wird. Diese Schwenkbewegung führt jedoch zwangsläufig auch das Abtriebsstirnrad 21 aus, welches an der Rotationsachse des Effektors 3 fixiert und mit dem Übertragungsstirnrad 22 in Kämmeingriff ist. Würde demzufolge das Übertragungsstirnrad 22 bei dieser Betätigungsart, d.h. der Verschwenkbetätigung, feststehen, würde die Schwenkbewegung des Instrumentenkopfs 3 ein gleichläufiges Abrollen des Abtriebsstirnrads 21 an dem Übertragungsstirnrad 22 bewirken und somit zwangsläufig zu einer überlagerten Rotationsbewegung des Effektors 3 führen.

Wie vorstehend jedoch ausgeführt wurde, wird die Zahnradwelle 26 bei einer Schwenkbewegung des Griffstücks 5 zusammen mit dem Kulissenelement 8 mitgedreht und treibt dabei die Antriebswelle 24 innerhalb des Schubrohrs 12 an. Die Übersetzung zwischen der Zahnradwelle 26 und der Antriebswelle 24 ist dabei so berechnet, dass das Übertragungszahnrad 22 durch die Anriebswelle 24 um einen solchen Drehwinkel gedreht wird, der dem Drehwinkel entspricht, welcher durch das Abtriebszahnrad 21 bei einer entsprechenden Abwinkelung des Instrumentenkopfs 4 hervorgerufen wird, wodurch sich beide Drehungen aufgrund ihrer Gegenläufigkeit kompensieren. Bei dieser Konstellation wird die Relativposition zwischen dem Übertragungsstirnrad 22 und dem Abtriebsstirnrad 21 auch während der Abwinkelungsbewegung des Instrumentenkopfs 4 beibehalten, so dass der Effektor 3 in jeder Abwinkelungsposition des Instrumentenkopfs 4 bzw. während einer Abwinkelungsbewegung in seiner augenblicklichen Rotationsposition bezüglich des Instrumentenkopfs 4 gehalten wird.

Wie vorstehend bereits ausgeführt wurde, kann die Kupplung 37, vorliegend in Form einer Rutschkupplung nur ein vorbestimmtes maximales Drehmoment übertragen. Dieses maximale Drehmoment ist dabei so gewählt, dass es im normalen Einsatz des Instruments nicht überschritten werden kann. Die maximale Abwinkelposition wie auch die maximale Streckposition des Instrumentenkopfs 4 wird durch einen Anschlagsmechanismus des Scharniers zwischen dem Instrumentenkopf 4 und dem Rohrschaft 2 definiert, wie dies vorstehend bereits beschrieben wurde. Sobald der Instrumentenkopf 4 diese Positionen erreicht hat, ist ein weiteres Abwinkeln des Instrumentenkopfs 4 in die entsprechende Richtung nicht mehr möglich.

Diese konstruktive Ausgestaltung läßt sich in vorteilhafter Weise zur vorbestimmbaren Einstellung der Relativposition des Griffstücks 5 bezüglich des Rohrschafts 2 d.h. zur sogenannten Nullpunkteinstellung ausnutzen. Zu diesem Zweck wird der Instrumentenkopf 4 in die eine oder andere maximale Anschlagsposition abgewinkelt. Nach die entsprechende Anschlagsposition erreicht ist übt die Bedienerperson eine zusätzliche Schwenkkraft auf den Instrumentengriff 1 aus, solange, bis das hieraus resultierende Drehmoment das maximal übertragbare Drehmoment der Kupplung 37 übersteigt. In diesem Augenblick gleitet der Anschlusssockel 39 innerhalb der Drehwelle 8 ab, wodurch sich die ürsprüngliche Winkelposition des Instrumentengriffs 1 bezüglich des Rohrschafts 2 für die ausgewählte maximale Anschlagsposition des Instrumentenkopfs 4 verändert.

Um eine Betätigung des Effektors 3 d.h. dessen Funktion selbst zu bewirken, ist in dem vorliegenden bevorzugten Ausführungsbeispiel der am Griffstück 5 schwenkbar gelagerte Hebel 7 vorgesehen. Wie bereits vorstehend zu den Fig. 6a-6c ausgeführt wurde, ist der Hebel 7 über ein nicht weiter dargestelltes Umlenkgetriebe oder einen entsprechenden Gelenkmechanismus mit dem in der Drehwelle 24 gelagerten Schubstab 35 wirkverbunden, welcher sich bei einer entsprechenden Betätigung des Hebels 7 relativ zur Drehwelle 24 axial hin- und herbewegt. Auch ein einfacher Bowdenzug oder ein Umlenkhebel wäre für eine Kraftübertragung auf den Schubstab 35 denkbar.

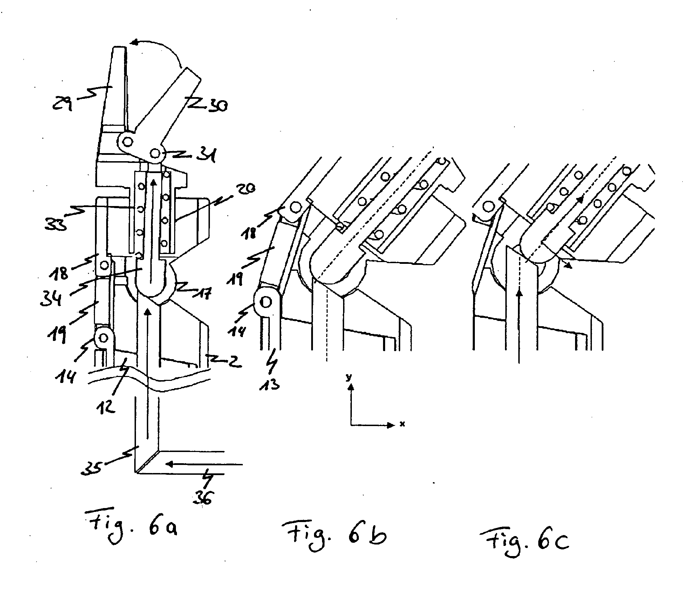

Die Fig. 6a zeigt nunmehr die Relativlage des Schubstabs 35 sowie des Schubbolzens 32 in 0°-Abwinkelposition des Instrumentenkopfs 4 bei geöffneter Zange, die Fig. 6b zeigt die Relativlage des Schubstabs 35 sowie des Schubbolzens 32 in ca. 45°-Abwinkel position des Instrumentenkopfs 4 bei geöffneter Zange und die Fig. 6c zeigt die Relativlage des Schubstabs 35 sowie des Schubbolzens 32 in ca. 45°-Abwinkelposition des Instrumentenkopfs 4 bei geschlossener Zange.

Wie aus den Fig. 6a-6c zu entnehmen ist, wird der Schubbolzen 32 durch die Vorspannkraft der Feder 33 in ständiger Anlage mit der abgeschrägten oder gefasten distalen Stirnfläche des Schubstabs 35 gehalten. Bei einer Verschiebung des Schubstabs 35 in Richtung Instrumentenkopf 4 im Fall einer 0°-Abwinkelung des Instrumentenkopfs 4 gemäß der Fig. 6a wird der Schubbolzen 32 mit gleicher Geschwindigkeit und Wegstrecke wie der Schubstab 35, d.h. ohne Übersetzung, entgegen der Vorspannkraft der Feder 33 verschoben, wodurch die daran angelenkte Zangenbacke 30 in Schließrichtung verschwenkt wird.

An dieser Stelle sei darauf hingewiesen, dass durch die Verschiebetätigkeit des Schubstabs 35 der Schubbolzen 32, d.h. insbesondere der Mittelpunkt des Bolzenkopfradius nur annähernd auf der Schwenkachse des Instrumentenkopfs 4 verbleibt, sich also bei einer Abwinkelungsbewegung des Instrumentenkopfs 4 auf einer Art Kreisbahn bewegt. Wie jedoch eingangs der Figurenbeschreibung bereits ausgeführt wurde, sind die Stellwege für ein Öffnen und Schließen beispielsweise der Zange aufgrund der eingestellten Übersetzungen so gering, dass der Kreisbahnradius zwar theoretisch berechenbar ist, jedoch bereits schon aus fertigungstechnischen Gründen (Eigenelastizität der verwendeten Materialien, Maßtoleranzen und Spiel an den Gelenkverbindungen und Getriebeteilen) keinen relevanten Einfuß auf Zangenstellung hat. In anderen Worten ausgedrückt, wird die Zangenstellung durch die Position des Hebels 7 bestimmt, welcher wiederum von einer Bedinerperson gehalten wird und damit beispielsweise auch unkontrollierbaren Handbewegungen (Zitterbewegungen) unterliegt. Derartige aufgrund manueller Betätigungen erzeugte Störungen sind im Vergleich zu jenen Störungen, welche durch die vorstehend beschriebene Kreisbahnbewegung erzeugt werden, wesentlich größer und daher praktisch alleinig maßgeblich.

D.h. unabhängig von der aktuellen Position des Schubstabs 35 bzw. des Schubbolzens 32 bewirkt eine Abwinkelung des Instrumentenkopfs 4 generell nicht nur ein Verschwenken des Schubbolzens 32 bezüglich des Schubstabs 35 sondern auch ein geringes Abgleiten des Bolzenkopfs 34 auf der abgeschrägten Stirnfäche des Schubstabs 35. Durch diese geringe Abgleitbewegung bleibt der Anlagekontakt des Schubbolzens 32 auf der Stirnfläche erhalten, wobei jedoch nur eine solche Ausgleichs-Längsbewegung des Schubbolzens 32 in Folge seiner Abgleitbewegung erfolgt, die zu keiner praktisch relevanten Veränderung der Schließ- oder Öffnungsposition am Effektor 3 führt. Gleichzeitig jedoch wird eine Art Kraftumlenkmechanismus geschaffen, um eine Längsbewegung des Schubstabs 35 in eine Längsbewegung des nunmehr im Winkel zum Schubstab 35 stehenden Schubbolzens 32 durch die Abschrägung der Schubstab-Stirnfläche zu bewirken.

In anderen Worten ausgedrückt, wird der Schubstab 35 bei einer Abwinkelungsposition > 0° gemäß der Fig. 6b in Schließrichtung des Effektors 3 verschoben, wie dies in der Fig. 6c dargestellt ist, gleitet die abgeschrägte Stirnfläche des Schubstabs 35 längs am Bolzenkopf 34 vorbei und übt dabei eine Vorschubkraft auf den Schubbolzen 32 aus, der sich dementsprechend in Schließrichtung des Effektors 3 bewegt.

## Patentansprüche

1. Chirurgisches Instrument mit einem Instrumentengriff (1), der an einem proximalen Endabschnitt eines Rohrschafts (2) angelenkt ist, an dessen distalem Endabschnitt ein Instrumentenkopf (4) abwinkelbar angelenkt ist, in dem ein Effektor (3) drehbar lagert, der zumindest ein schwenkbares Eingriffselement (30) hat, wobei der Instrumentengriff (1) eine Anzahl von Handhaben und/oder Betätigungsmechanismen zur Betätigung des Instrumentenkopfs (4) und/oder des Effektors (3) über Getriebezüge aufweist,
**dadurch gekennzeichnet, dass**
in dem die Abwinkelbewegung des Instrumentenkopfs (4) bewirkenden Getriebezug eine Kupplung (37) zwischengefügt ist, die eine zumindest über einen vorbestimmten Schwenkbereich des Instrumentengriffs (1) individuelle Nullpunkteinstellung erlaubt, wobei der Nullpunkt eine Relativposition des Instrumentengriffs (1) bezüglich des Rohrschafts (2) ist, in der der Instrumentenkopf (4) eine vorbestimmte Abwinkelposition bezüglich des Rohrschafts (2) einnimmt.

2. Chirurgisches Instrument nach Anspruch 1, **dadurch**
**gekennzeichnet, dass**
die vorbestimmte Abwinkelposition die maximale oder minimale Abwinkelposition des Instrumentenkopfs (4) ist.

3. Chirurgisches Instrument nach Anspruch 1 oder 2,
**dadurch gekennzeichnet, dass**
die Kupplung (37) eine Rutschkupplung mit einem vorbestimmten maximal übertragbaren Drehmoment ist.

4. Chirurgisches Instrument nach einem der vorstehenden Ansprüche, **dadurch gekennzeichnet, dass**
die Kupplung (37) im Anlenkbereich zwischen dem Instrumentengriff (1) und dem Rohrschaft (2) angeordnet ist.

5. Chirurgisches Instrument nach Anspruch 4, **dadurch**
**gekennzeichnet, dass**
die Kupplung (37) aus einem am Instrumentengriff (1) fest angeordneten Anschlussstutzen (39) und einer am Rohrschaft (2) für die Übertragung einer Abwinkelbewegung auf den Instrumentenkopf (4) drehbar gehaltenen Drehwelle (8) besteht, die reibschlüssig mit dem Anschlussstutzen (39) verbunden ist.

6. Chirurgisches Instrument nach Anspruch 5, **dadurch**
**gekennzeichnet, dass**
die Drehwelle (8) sowie der Anschlussstutzen (39) zumindest abschnittsweise ineinandergesteckt sind, wobei zwischen der Drehwelle (8) und dem Anschlussstutzen ein Reibelement (38) angeordnet ist.

7. Chirurgisches Instrument nach Anspruch 6, **dadurch**
**gekennzeichnet, dass**
das Reibelement (38) ein offener Federring ist, der auf einer inneren und/oder äußeren Umfangsgleitfläche an der Drehwelle (8) und/oder dem Anschlussstutzen (39) unter Vorspannung aufgesetzt ist und damit eine Reibschlußverbindung mit der Drehwelle (8) und/oder dem Anschlussstutzen (39) zur Übertragung eines über die Federvorspannkraft vorbestimmten Drehmoments eingeht.

8. Chirurgisches Instrument nach Anspruch 7, **gekennzeichnet durch** einen Mitnehmerstift (40), der entweder in die Drehwelle (8) oder den Anschlussstutzen (39) radial eingesetzt ist und in einen vom offenen Federring (38) gebildeten Radialspalt ragt.

## Claims

1. A surgical instrument having an instrument handle (1) joined to a proximal end portion of a tubular shank (2), to the distal end portion of which an instrument head (4) is joined so as to be bendable, in which instrument head (4) there is rotatably mounted an effector (3), which has at least one swivellable engaging element (30), the instrument handle (1) having a number of manual control means and/or actuating mechanisms for actuating the instrument head (4) and/or the effector (3) via gear trains,
**characterised in that**,
in the gear train effecting the bending movement of the instrument head (4), there is interposed a clutch (37) which allows individual zero point adjustment at least over a predetermined swivel range of the instrument handle (1), wherein the zero point is a relative position of the instrument handle (1) with regard to the tubular shank (2) in which the instrument head (4) assumes a predetermined bent position relative to the tubular shank (2).

2. A surgical instrument according to claim 1, **characterised in that** the predetermined bent position is the maximum or minimum bent position of the instrument head (4).

3. A surgical instrument according to claim 1 or claim 2, **characterised in that** the clutch (37) is a slip clutch with a predetermined maximum transmittable torque.

4. A surgical instrument according to any one of the preceding claims, **characterised in that** the clutch (37) is arranged in the area in which the instrument handle (1) is joined to the tubular shank (2).

5. A surgical instrument according to claim 4, **characterised in that** the clutch (37) consists of a connecting piece (39) arranged fixedly on the instrument handle (1) and a rotary shaft (8) mounted rotatably on the tubular shank (2) for transmission of a bending movement to the instrument head (4), said rotary shaft (8) being connected frictionally with the connecting piece (39).

6. A surgical instrument according to claim 5, **characterised in that** the rotary shaft (8) and the connecting piece (39) are fitted one inside the other at least in part, wherein a friction member (38) is arranged between the rotary shaft (8) and the connecting piece.

7. A surgical instrument according to claim 6, **characterised in that** the friction member (38) is an open spring washer, which is positioned under bias on an inner and/or outer circumferential sliding surface on the rotary shaft (8) and/or the connecting piece (39) and thus enters into frictional engagement with the rotary shaft (8) and/or the connecting piece (39) for transmitting a torque predetermined by means of the spring bias force.

8. A surgical instrument according to claim 7, **characterised by** a driver pin (40), which is inserted radially either into the rotary shaft (8) or the connecting piece (39) and projects into a radial gap formed by the open spring washer (38).

## Revendications

1. Instrument chirurgical avec poignée (1), qui est articulée au niveau d'une section d'extrémité proximale d'une tige tubulaire (2), sur la section d'extrémité distale de celle-ci est articulée une tête d'instrument (4) de manière à pouvoir se plier, dans laquelle un effecteur est disposé de manière à pouvoir tourner, qui présente au moins un élément de préhension (30) pouvant pivoter, la poignée (1) présentant une pluralité de mécanismes d'actionnement et/ou de manipulation pour actionner la tête de l'instrument (4) et/ou l'effecteur (3) par des trains d'engrenages,
**caractérisé en ce que**
un dispositif d'accouplement (37) est ajouté dans le train d'engrenages qui produit le mouvement de pliage de la tête de l'instrument (4), lequel dispositif permet un réglage à un point zéro individuel au moins par une zone de pivotement prédéterminée de la poignée (1), le point zéro est une position relative de la poignée (1) par rapport à la tige tubulaire (2), dans laquelle la tête de l'instrument (4) occupe une position de pliage prédéterminée par rapport à la tige tubulaire (2).

2. Instrument chirurgical selon la revendication 1,
**caractérisé en ce que**
la position de pliage prédéterminée correspond à la position de pliage maximale ou minimale de la tête (4) de l'instrument.

3. Instrument chirurgical selon la revendication 1 ou 2,
**caractérisé en ce que**
le dispositif de couplage (37) est un accouplement par glissement avec un moment de couple prédéterminé transmissible au maximum.

4. Instrument chirurgical selon l'une quelconque des revendications précédentes, **caractérisé en ce que**
le dispositif d'accouplement (37) est disposé dans la zone d'articulation entre la poignée (1) et la tige tubulaire (2).

5. Instrument chirurgical selon la revendication 4,
**caractérisé en ce que**
le dispositif d'accouplement (37) comprend un embout de raccordement (39) disposé solidement sur la poignée (1) et un arbre rotatif (8) maintenu en rotation sur la tige tubulaire (2) pour transférer le mouvement d'articulation à la tête (4) de l'instrument, lequel arbre rotatif entraîné par friction, est relié à l'embout de raccordement (39).

6. Instrument chirurgical selon la revendication 5,
**caractérisé en ce que**
l'arbre rotatif (8) ainsi que l'embout de raccordement (39) sont au moins de manière partielle introduits l'un dans l'autre, un élément de friction (38) est disposé entre l'arbre rotatif (8) et l'embout de raccordement.

7. Instrument chirurgical selon la revendication 6,
**caractérisé en ce que**
l'élément de friction (38) est une rondelle-ressort ouverte, qui est montée par précontrainte sur une surface de glissement périphérique interne et/ou externe sur l'arbre rotatif (8) et/ou sur l'embout de raccordement (39) et ce qui permet une liaison de fermeture par friction avec l'arbre rotatif (8) et/ou avec l'embout de raccordement (39) pour transférer un moment de couple prédéterminé par la force de la tension du ressort.

8. Instrument chirurgical selon la revendication 7,
**caractérisé en ce qu'**
une broche d'entraînement (40), qui est insérée soit dans l'arbre rotatif (8) soit dans l'embout de raccordement (39) de manière radiale et dépasse dans une fente radiale formée par la rondelle-ressort ouverte (38).
